# EUROPEAN PATENT SPECIFICATION

(11) **EP 2 515 956 B1**
(45) Date of publication and mention of the grant of the patent: **16.03.2016**
(21) Application number: 10794980.2
(22) Date of filing: 15.12.2010
(51) Int. Cl.: A61L 15/46, A61K 9/00, A61K 33/00, C01G 23/047, A61K 9/14

(54) **COMPOSITION COMPRISING NANOPARTICLES OF TiO2**
ZUSAMMENSETZUNG MIT TIO2-NANOPARTIKELN
COMPOSITION COMPORTANT DES NANOPARTICULES DE TIO2

(30) Priority: 15.12.2009 US 286464 P; 15.12.2009 SE 0950965
(43) Date of publication of application: 31.10.2012
(73) Proprietor: Universitetet I Oslo, 0316 Blinden (NO)
(72) Inventor: TAXT-LAMOLLE, Sébastien Francis Michel, N-0375 Oslo (NO); LYNGSTADAAS, S., Petter, N-1450 Nesoddtangen (NO); HAUGEN, Håvard, J., N-0376 Oslo (NO)
(74) Representative: Valea AB
(86) International application number: PCT/EP2010/069808
(87) International publication number: WO 2011/080080

(56) References cited:
- EP-A1- 1 192 933
- WO-A1-89/06548
- WO-A1-2008/103082

## Description

### Technical field

The present invention is in the field of wound care products. More particularly it relates to an antimicrobial and/or anti-inflammatory composition comprising activated nanoparticles of TiO₂, which composition does not cause microbial resistance.

### Background art

Modern wound dressings (Lait et al., Journal of clinical nursing, 7, (1998) 11-7, Bishop, Critical care nursing clinics of North America, 16, (2004) 145-77, Jones, International wound journal, 3, (2006) 79-86) include gauzes (which may be impregnated with an agent designed to help sterility or to speed healing), films, gels, foams, hydrocolloids, alginates, hydrogels and polysaccharide pastes, granules and beads. Materials typically used are polymer-based, such as polyamides, silicones, high density polyethylene, polyester, polypropylene, polyurethane, polysulphone. A dressing can have a number of purposes, depending on the type, severity and position of the wound, although all purposes are focused towards promoting recovery and preventing further harm from the wound. Key purposes of wound dressings are:
1) Stem bleeding - Helps to seal the wound to expedite the clotting process
2) Absorb exudates - Soak up blood, plasma and other fluids exuded from the wound, containing it in one place
3) Ease pain - Some dressings may have a pain relieving effect, and others may have a placebo effect
4) Debridement of the wound - The removal of slough and foreign objects
5) Protection from infection, inflammation and mechanical damage, and
6) Promote healing - through granulation and epithelialisation

Part 5 is the current problem in wound healing today. The microbiology of most wound types is complex, involving both aerobic and anaerobic bacteria (Gilchristet et al., The British journal of dermatology, 121, (1989) 337-44, Mousa, The Journal of hospital infection, 37, (1997) 317-23, Bowler et al., The Journal of burn care & rehabilitation, 25, (2004) 192-6) and these organisms can create a potential problem to both the wound in which they reside (i.e. autoinfection) and the surrounding environment (cross-contamination). This is in particularly relevant to patients with wounds that has (1) increasing signs of bacterial influence, (2) increasing odour, pain or exudates, (3) redness, (4) signs of pseudomonas, (5) oedema, (6) the healing which does not progress normally and/or (7) increased skin temperature. Patients with low to moderately exuding wounds, such as leg and foot ulcers, pressure ulcers and partial thickness burns, are also particularly susceptible to these indications. If one can modulate local inflammatory responses and hinder bacterial (re)colonization in the wound without disturbing the healing process, a significant step forward would be achieved in modern wound care.

One of the key approaches for minimising the likelihood of serious wound infections is the use of topical antimicrobial agents. The purpose of these antimicrobial agents is to reduce the microbial load (bioburden) in wounds, and hence the opportunity for infection. Typically these have involved (Bowler, Jones, The Journal of burn care & rehabilitation, 25, (2004) 192-6) the use of broad spectrum antiseptic agents (e.g. iodine and silver) and antibiotics (e.g. neomycin, bacitracin and polymyxin combinations). However, due to problems with the development of bacterial resistance to antibiotics, the search for alternative substances having an antimicrobial effect continues.

Currently, the medical device industries instead of antibiotics often incorporate silver (Ag-ions) into wound dressings in order to achieve an antibacterial effect. The silver ions are released and penetrate deep into the wound. Silver ions released by such dressings offer advantages in suppressing infection and bacterial toxins (Lansdown AB 2004 A review of the use of silver in wound care: facts and fallacies Br J Nurs 13 S6-19). Unlike antibiotics, that usually target one cell function, the efficacy of silver releasing dressing is based on interference with microbial proliferation through altering DNA and RNA, causing fatal structural changes in bacterial cell walls and membranes (Bolton L 2006 Are silver product safe and effective for chronic wound management? J Wound Ostomy Continence Nurs 33 469-77). It is hypothesized that these silver ions reduce the deeper-laying bacterial burden. However, the use of silver ions in wound care products have the serious disadvantage that patients with silver sensitivity may have an allergic response. Additional drawbacks are that it cannot be used during radiation treatment or X-ray examinations, ultrasound, diathermy or Magnetic Resonance Imaging or together with oxidising agents, such as hypochlorite solutions or hydrogen peroxide. The use of silver may also scar tissue and has a tattooing effect, turning the skin close to the healing wound blue. In addition, the antibacterial effect of silver containing wound care products is disputed (Chaby G et al. 2007 Dressing for acute and chronic wounds; a systematic review Arch Dermatol 143 1297-304, Lo SF et al. A systematic review of silver-releasing dressing in the management of infected chronic wounds J Clin Nurs 17 1973-85). Also, the use of silver ions have the disadvantage that the silver ions remain toxic, which renders the use of them environmentally hazardous as the ions never stop exerting their toxic effect on bacteria. Wound dressing with silver are also costly to manufacture and has not provided predictable results so far. Hence, there exists a need within this field to provide an improved wound dressing which solves the problems associated with the wound dressings available today.

TiO₂, titanium (IV) oxide or titania is the naturally formed oxide of titanium and a very well-known and well-researched material due to the stability of its chemical structure, its biocompatibility, and physical, optical and electrical properties. Titanium dioxide occurs in nature as the well-known naturally occurring minerals rutile, anatase and brookite. It has previously been disclosed that TiO₂, when activated by UV-light, forms the free radical TiO-OH⁻. UV-activated TiO₂ is a promising technique for decontaminaton, purification, and deodorization of air and wastewater (Yu *et al.* 2001, Hoffman *et al.*. 1995, Ollis *et al.* 1985, Fox *et al.* 1993 and Somorjai *et al.* 1996) and has also been applied to inactive bacteria, viruses and cancer cells (Hur *et al.* 2002, Maness et al. 199, Yu *et al.* 2003, Sunada *et al.* 2003). Also, TiO-OH- is disclosed to have an anti-fouling and antibacterial effect (Byrne et al. 1998, Hur *et al.* 2002, Maness *et al.* 1999, Yu et al. 2001). Each of WO 2008/019869 and WO 2008/103082 discloses a composition comprising nanoparticles of TiO₂ having a size of 1 nm -1 µm which composition also comprises hydrogen peroxide. The composition is activated by UV light and used for the treatment of superficial fungal, bacterial and/or viral disorders. Due to the use of UV light, this composition is not suitable for use for the treatment of deep wounds as the UV light has difficulties to reach and activate the TiO₂ particles deep down in wounds. Furthermore, the use of UV-light to activate TiO₂ is not always convenient when it comes to products for medical use, due to the risk for skin burns and risk for degradation of wound dressing polymers by the UV-light.

It has also been previously shown that ceramic TiO₂ may be activated to form the free radical TiO-OH- in the presence of H₂O₂. Silva et al. (Silva et al. 2007 Effect of key operating parameters on phenols degradation during H2O2-assisted TiO2 photocatalytic treatment of simulated and actual olive mill wastewaters Appl Catalysis 73 11-22) used nanoparticles of TiO₂ for treatment of olive mill wastewater by activating the TiO₂ particles by using a combination of very low amounts of H₂O₂ (up to 0.5 mM) and UV-light.

In WO 89/06548 it is disclosed that the reaction product of H₂O₂ and metallic titanium, a gel, may be used for anti-inflammatory purposes. The gel is described as acting a slow release H₂O₂ reservoir.

SE 531319 C2 discloses granules comprising titanium, TiO₂ or alloys of titanium with TiO₂ on their surface. The granules have an average length from one side to the other of between 200 micrometers up to 5 mm and are disclosed to have anti-inflammatory and antibacterial properties. It is pointed out in SE 531319 that it is important that the particles do not have a size less than 5 micrometers, as the granules then will be phagocytized by macrophages, resulting in the particles not any more being able to exert their anti-inflammatory and antibacterial properties.

Even with the development in wound care during the last years there still exists a need for improved wound dressings that can aid in wound healing by the prevention or treatment of microbial infections or that reduce inflammatory reactions without negative side effects.

### Summary of the invention

The object of the present invention is to provide an improved composition according to any of claims 1-15 having antimicrobial and/or anti-inflammatory properties, in particular for use for wound debridement.

This object is solved by the provision of a composition comprising nanoparticles of TiO₂ having a mean particle diameter (D₅₀) of about 3-150 nm at a concentration of about 1-2000 g/L and H₂O₂ at a final concentration at about 2.5-25 % by volume. The TiO₂ particles in the composition are activated by the H₂O₂ to form radical species, such as Ti-OH⁻, Ti- µ-peroxide and Ti-η²-peroxide on the surface of the nanoparticles Preferably, the mean particle diameter (D₅₀) of the nanoparticles is about 20-50 nm in such a composition.

The present invention therefore is directed to a composition obtainable by mixing nanoparticles of TiO₂ having a mean particle diameter (D₅₀) of about 3-150 nm to a concentration of about 1-2000 g/L and H₂O₂ to a final concentration at about 2.5-25 % by volume. This composition comprises activated TiO₂ nanoparticles. Preferably, the mean particle diameter (D₅₀) of the nanoparticles is about 20-50 nm in such a composition. Another aspect of the invention is directed to the composition for medical use.

The invention is also directed to the use of the composition for the manufacture of a pharmaceutical composition for preventing microbial growth, for wound debridement, for locally alleviating inflammation, and/or for simultaneously preventing microbial growth and debriding a wound. The invention is also directed to the composition for use for preventing microbial growth, for wound debridement, for locally alleviating inflammation, and/or for simultaneously preventing microbial growth and debriding a wound.

Another aspect of the invention is directed to a method for preventing microbial growth a wound, debriding a wound, treating and/or alleviating local inflammations, and/or simultaneously preventing microbial growth and debriding in and/or around a wound comprising the step of administering the composition to a subject in need thereof.

The invention is also directed to medical products or devices and/or cosmetic products or devices comprising the composition.

Yet another aspect of the invention is directed to a kit comprising a first container comprising TiO₂ having a mean particle diameter (D₅₀) of about 3-150 nm and a second container comprising H₂O₂, which, when mixed with each other, provide the composition. The invention is also directed to a method for preparing the composition comprising the steps of mixing said nanoparticles of TiO₂ with said H₂O₂. Preferably the mean particle diameter (D₅₀) in such a kit is 20-50 nm.

### Definitions

### Brief description of drawings

Figure 1: Methylene blue degradation with different concentrations of TiO₂ in a solution of 15% H₂O₂.
Figure 2: Methylene blue degradation with different concentrations of H₂O₂ in a solution of TiO₂ at 0.5 g/L.
Figure 3: Methylene blue degradation when mixed with a suspension containing H₂O₂ at 5% and TiO₂ at 1.6 g/L, and doped with 1.6 g/L NaCl and 1.6 g/L NaF.

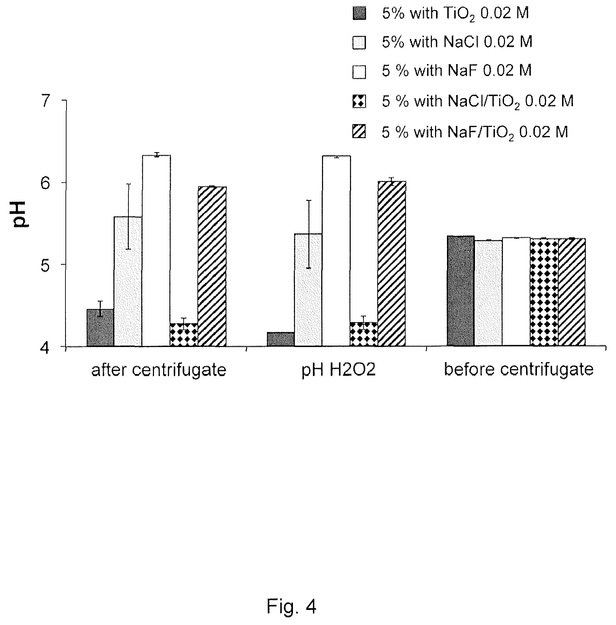

Figure 4: The pH of the suspensions change depending on the composition of the suspension.
Figure 5: Spectrophotometry analysis of the biomass measured through safranin staining after exposure of the inoculated samples to the four cleaning solutions for 2 minutes.

### Detailed description of invention

The present invention is directed to a composition comprising nanoparticles of TiO₂ and H₂O₂. The composition is antimicrobial and may inhibit bacterial growth. It is also anti-inflammatory and/or anti-fouling and is particularly useful for the debridement of wounds to remove organic debris, microbes and/or to alleviate inflammation in and around the wound and/or for the prevention of growth of microorganisms. Also, the composition of the invention has an immunomodulatory effect. The composition is therefore useful for cleaning wounds to improve their healing. Due to its beneficial pharmaceutical properties, the composition of the invention may also be denoted as a pharmaceutical composition or an antibacterial, anti-fouling and/or anti-inflammatory composition.

A first aspect of the invention is directed to a composition comprising nanoparticles of TiO₂ having a mean particle diameter (D₅₀) of about 3-150 nm at a concentration of about 1-2000 g/L and H₂O₂ at a final concentration at about 2.5-25 % by volume. The concentrations of TiO₂ particles and H₂O₂, respectively, as specified for the compositions herein, refer to their respective final concentrations in the compositions. The mean particle diameter (D₅₀) of the TiO₂ nanoparticles is preferably about 20-50 nm. When the nanoparticles of TiO₂ are mixed with the H₂O₂, the surface of the particles is activated and radicals such as Ti-OH⁻, Ti- µ-peroxide and Ti-η²-peroxide are formed on the surface[25]. The composition of the invention may therefore also be described as a composition comprising activated nanoparticles of TiO₂, meaning the presence of any one of the Ti-OH⁻, Ti- µ-peroxide and Ti-η²-peroxide radicals or mixture of one or more of these on the surface of the particles. Preferably the composition of the invention consists of the nanoparticles of TiO₂ and H₂O₂ at the respective sizes and concentrations as defined herein.

Compared to other antimicrobial agents, TiO₂ is particularly suitable for pharmaceutical use such as wound treatment due to its properties such as stability, environmental safety, broad spectrum antibiosis and anti-inflammatory properties. As mentioned above, when a particle comprising TiO₂ is subjected to UV-light or H₂O₂ an activated, free radical Ti-OH⁻, Ti- µ-peroxide and Ti-η²-peroxide particle is formed. The compositions of the invention may therefore also be denoted as compositions comprising activated nanoparticles of TiO₂, i.e. nanosized Ti-OH⁻, Ti-µ-peroxide and/or Ti-η²-peroxide particles, wherein "activated" means that the formation of free radicals of at least part of the TiO₂ particles has taken place. The formation of radicals takes place on the surface of the TiO₂ nanoparticle, so it is (at least part of) the surface of the TiO₂ nanoparticle that is "activated". The present invention is therefore also directed to a composition obtainable by the mixing of nanoparticles of TiO₂ having a mean particle diameter (D₅₀) of about 3-150 nm so that the final concentration of said nanoparticles of TiO₂ is 1-2000 g/L and H₂O₂ so that the final concentration of H₂O₂ is about 2.5-25 % by volume. The mean particle diameter (D₅₀) of the TiO₂ nanoparticles is preferably about 20-50 nm. For this aspect of the invention, the TiO₂ nanoparticles and the H₂O₂ may also be mixed so as to provide the preferred concentrations of TiO₂ and H₂O₂, respectively, according to the preferred compositions disclosed herein.

As mentioned above, TiO₂ particles may be activated by UV-light to form the radicals. However, the use of H₂O₂ to activate the nanoparticles instead has a number of advantages. Firstly, the use of H₂O₂ avoids the need for an extra device, the UV-lamp. Also, by the addition of H₂O₂ to the TiO₂ nanoparticles the OH- formation is accelerated leading to improved reaction activity [16-19]. Additionally high exposure of UV-light might cause skin burns and even degrade the wound dressing's polymer. These degradation products typically cause inflammation and disrupt healing. These disadvantages are avoided when H₂O₂ is used to activate the TiO₂ nanoparticles. Also, the H₂O₂ has advantageous properties in itself when it comes to treatment of wounds in order to debride them and prevent microbial growth. Hydrogen peroxide has since long been used as an antiseptic and anti-bacterial agent due to its oxidizing effect. When used for wound treatment, hydrogen peroxide causes mild damage to tissue but it also may stop capillary bleeding and clean the wound.

The present inventors have surprisingly found that by using the specific concentration intervals and sizes of nanoparticles of TiO₂, a particularly high amount of the nanoparticles are activated and a particularly high amount of the Ti-OH⁻, Ti- p-peroxide and Ti-η²-peroxide radicals are formed. When UV light is used to active the TiO₂ particles, the size of the particles is not at all as critical. However, the present inventors have found that when H₂O₂ is used to activate the surface of the TiO₂ nanoparticles, it is important to select nanoparticles having the mean particle diameters specified herein, in order to obtain a good surface reaction and formation of radicals on the surface of the TiO₂ particles. A particularly good surface reaction is obtained when the mean particle diameter of the TiO₂ nanoparticles is about 20-50 nm. Due to the small size of the TiO₂ nanoparticles, they have a large surface-to-volume ratio and thereby a higher antimicrobial, anti-inflammatory and/or debriding effect. The present inventors have surprisingly found that at the range of mean particle diameter of the TiO₂ nanoparticles in the composition of the invention, a particularly high reaction between the TiO₂ nanoparticles and the H₂O₂ takes place, which leads to a particularly large formation of radicals. This effect is particularly pronounced when the TiO₂ nanoparticles have a mean particle diameter (D₅₀) of about 20-50 nm, such as about 20 nm, 25 nm, 30 nm, 35 nm, 40 nm, 45 nm or 50 nm. Also, the composition of the invention, i.e. a composition having the specified ratio of concentration of TiO₂ and H₂O₂ and the particular size range of the particles, provides a composition with a consistency that is very suitable for application to a wound. The composition of the invention is therefore particularly effective when used as a pharmaceutical composition e.g. in wound treatment, such as for wound debridement and/or for the prevention of microbial growth since one cannot use any stronger concentration than 7.5% by volume of H₂O₂, normally only 5% by volume, of H₂O₂ when contact with living tissue. It is therefore impossible to increase the anti-microbial effect with a higher H₂O₂ concentration. However, by the use of the composition of the present invention comprising nanoparticles of TiO₂ and H₂O₂, it is possible to use a higher concentration of H₂O₂ in the composition than normally can be applied to living tissue if H₂O₂ is used alone.

In the below, "nanoparticles of TiO₂" is often used to denote particles of TiO₂ having a mean particle diameter (D₅₀) of about 3-150 nm. By "nanoparticles" is in the present context meant particles having a mean particle diameter (D₅₀) between about 3 and 150 nm.

The concentration of TiO₂ in compositions of the invention is preferably about 3-10 g/L, more preferably about 4-8 g/L.

In a composition of the invention the concentration of H₂O₂ is preferably about 3-25 % by volume, more preferably about 4-20 % by volume, yet more preferably about 5-15 % by volume.

The TiO₂ particles in a composition of the invention are of nanosize and have a mean particle diameter (D₅₀) of about 3-150 nm. Preferably, the nanoparticles of TiO₂ have a mean particle diameter (D₅₀) of about 20-50 nm, such as about 20, 25, 30, 35, 40, 45 or 50 nm. The nanoparticles may therefore have a mean particle diameter (D₅₀) of preferably about 20-30 nm, such as 20, 21, 22, 23, 24, 25, 26, 27, 28, 29 and 30 nm. A composition of the invention may comprise a mixture of TiO₂ nanoparticles of different sizes as long as the mean particle diameter is between about 3-150 nm, 20-50 nm or 20-30 nm.

A preferred composition of the invention comprises nanoparticles of TiO₂ having a mean particle diameter (D₅₀) of about 5-100 nm at a concentration of at least 1 g/L and H₂O₂ at a final concentration of about 2.5-7.5 % by volume. Another preferred composition of the invention comprises nanoparticles of TiO₂ having a mean particle diameter (D₅₀) of about 20-30 nm at a concentration of about 3-10 g/L and H₂O₂ at a final concentration of about 3-20% by volume. Another preferred composition of the invention comprises nanoparticles of TiO₂ having a mean particle diameter (D₅₀) of about 20-30 nm at a concentration of about 4-8 g/L and H₂O₂ at a final concentration of about 5-15 % by volume.

In particular, the present invention is directed to a composition comprising nanoparticles of TiO₂ having a mean particle diameter (D₅₀) of about 20-50 nm at a concentration of about 1-2000 g/L and H₂O₂ at a final concentration at about 2.5-25 % by volume. Preferably in such a composition, the concentration of the TiO₂ nanoparticles is about 3-10 g/L, preferably about 4-8 g/L. The concentration of H₂O₂ in such a composition is preferably about 3-25 % by volume, preferably about 4-20 % by volume, more preferably about 5-15 % by volume. The mean particle diameter of the particles in such a composition may e.g. be about 20-30 nm, such as 20, 21, 22, 23, 24, 25, 26, 27, 28, 29 and 30 nm.

The invention is also directed to a composition comprising nanoparticles of TiO₂ having a mean particle diameter (D₅₀) of about 20-50 nm at a concentration of at least 1 g/L and H₂O₂ at a final concentration of about 2.5-7.5 % by volume.

Another preferred composition of the invention comprises nanoparticles of TiO₂ having a mean particle diameter (D₅₀) of about 20-50 nm at a concentration of about 3-10 g/L and a concentration of H₂O₂ of 3-25 % by volume.

Another preferred composition of the invention comprises nanoparticles of TiO₂ having a mean particle diameter (D₅₀) of about 20-50 nm at a concentration of about 3-10 g/L and a concentration of H₂O₂ of 4-20 % by volume.

Another preferred composition of the invention comprises nanoparticles of TiO₂ having a mean particle diameter (D₅₀) of about 20-50 nm at a concentration of about 4-8 g/L and a concentration of H₂O₂ of 3-25 % by volume.

A preferred composition of the invention comprises nanoparticles of TiO₂ having a mean particle diameter (D₅₀) of about 20-50 nm at a concentration of about 4-8 g/L, and hydrogen peroxide at a concentration of about 5-15 % by volume.

Another preferred composition of the invention comprises nanoparticles of TiO₂ having a mean particle diameter (D₅₀) of about 20-50 nm at a concentration of about 10-50 g/L, and hydrogen peroxide at a concentration of about 5-25 % by volume.

Another preferred composition of the invention comprises nanoparticles of TiO₂ having a mean particle diameter (D₅₀) of about 20-50 nm at a concentration of about 20-50 g/L, and hydrogen peroxide at a concentration of about 8-25 % by volume.

Another preferred composition of the invention comprises nanoparticles of TiO₂ having a mean particle diameter (D₅₀) of about 20-50 nm at a concentration of about 20-40 g/L, and hydrogen peroxide at a concentration of about 8.5-23 % by volume.

Another preferred composition of the invention comprises nanoparticles of TiO₂ having a mean particle diameter (D₅₀) of about 20-50 nm at a concentration of about 50-200 g/L, and hydrogen peroxide at a concentration of about 5-25 % by volume.

Another preferred composition of the invention comprises nanoparticles of TiO₂ having a mean particle diameter (D₅₀) of about 20-50 nm at a concentration of about 50-150 g/L, and hydrogen peroxide at a concentration of about 9-25 % by volume.

Another preferred composition of the invention comprises nanoparticles of TiO₂ having a mean particle diameter (D₅₀) of about 20-50 nm at a concentration of about 70-100 g/L, and hydrogen peroxide at a concentration of about 10-25 % by volume.

Another preferred composition of the invention comprises nanoparticles of TiO₂ having a mean particle diameter (D₅₀) of about 20-50 nm at a concentration of about 200-1000 g/L, and hydrogen peroxide at a concentration of about 5-25 % by volume.

Another preferred composition of the invention comprises nanoparticles of TiO₂ having a mean particle diameter (D₅₀) of about 20-50 nm at a concentration of about 400-800 g/L, and hydrogen peroxide at a concentration of about 11-25 % by volume.

Another preferred composition of the invention comprises nanoparticles of TiO₂ having a mean particle diameter (D₅₀) of about 20-50 nm at a concentration of about 500-700 g/L, and hydrogen peroxide at a concentration of about 13-25 % by volume.

The nanoparticles of TiO₂ in a composition of the invention may also have 0.01-5 atomic weight % fluorine in the surface layer, i.e. fluorine doped nanoparticles of TiO₂. The presence of fluorine on the surface of the TiO₂ nanoparticles further increases the particles' beneficial properties for wound treatment, i.e. a stronger antimicrobial and/or anti-inflammatory effect occurs. The fluorine may be added to the surface of the TiO₂ nanoparticles either before mixing with H₂O₂ or the fluorine may be present in the solution when the nanoparticles of TiO₂ are mixed with H₂O₂. In order to pre-treat nanoparticles of TiO₂, the particles may e.g. be subjected to an about 2-3 vol% fluorine solution, such as NaF or HF solution. Alternatively, NaF or HF may be present in the composition comprising the nanoparticles of TiO₂ and H₂O₂ at a concentration of 0.05-0.5 % by weight. Nanoparticles of TiO₂ pre-treated with fluorine are also commercially available e.g. from NaF (sodium fluoride, Sigma Aldrich, Oslo, Norway), HF (hydrofluoric acid, Sigma Aldtrich, Oslo, Norway), NH₄F (ammonia fluoride, Sigma Aldrich, Oslo, Norway) and/or any combination of the above.

A composition of the invention may also comprise further pharmaceutically acceptable agents. For example, the composition may comprise one or more emulsifiers, such as polyoxyethylene (20) sorbitan monolaurate, polyoxyethylene (20) sorbitan monopalmitate, polyoxyethylene (20) sorbitan, polyoxyethylene (20) sorbitan monooleate, PEG-ylated derived sorbitan and/or non-PEG-ylated) sorbitan and/or any combination of thereof. The composition may also further comprise one or more humectants, such as glycerine, propylene glycol (E 1520) and glyceryl triacetate (E1518), polyols like sorbitol (E420), xylitol and maltitol (E965), polymeric polyols like polydextrose (E1200), or natural extracts like quillaia (E999), lactic acid, urea and/or any combination of thereof. The composition may also further comprise one or more a gelling agent such as alginic acid (E400), sodium alginate (E401), potassium alginate (E402), ammonium alginate (E403), calcium alginate (E404), agar (E406, a polysaccharide obtained from red seaweeds), carrageenan (E407, a polysaccharide obtained from red seaweeds), locust bean gum (E410, a natural gum from the seeds of the Carob tree), pectin (E440, a polysaccharide obtained from apple or citrus-fruit), gelatin (E441, made by partial hydrolysis of animal collagen), polyoxyethylene polyoxypropylene block copolymer and/or any combination of thereof.

Although all three mineral forms of TiO₂, anatase, rutile and brookite, are possible to use for the purposes of the present invention, preferably the TiO₂ is predominantly in the anatase form. By predominantly is meant that at least 50% or the TiO₂ nanoparticles are in anatase form. More preferably, at least 60% of the TiO₂ nanoparticles are in the anatase form, even more preferably at least 70%, yet more preferably at least 80%. Also, at least 85%, such as 90 or 95%, of the TiO₂ may be in anatase form. The remainder of the TiO₂ may be in rutile and/or brookite form.

The composition of the invention is prepared by mixing said nanoparticles of TiO₂ with said H₂O₂ in such amounts that the resulting composition has the desired concentrations of TiO₂ and H₂O₂, respectively. The TiO₂ particles may be provided as a solid or in an aqueous suspension. In order to provide compositions with fluorine doped nanoparticles of TiO₂, NaF or HF may further be added in an amount so that the resulting concentration thereof in the composition is 0.05-0.5 % by weight. Consequently, the invention is also directed to a method for preparing a composition as disclosed herein, comprising the steps of mixing said nanoparticles of TiO₂ with said H₂O₂, optionally also comprising the step of adding NaF or HF. The invention is also directed to a composition obtainable by mixing said nanoparticles of TiO₂ having the desired mean particle diameter (D₅₀) with said H₂O₂. Interestingly, it has been noted that when nanoparticles of TiO₂ are mixed with increasing concentrations of H₂O₂ (within the above stated ranges of H₂O₂ concentration) a concomitant decrease in pH does not occur, although the antimicrobial effect still is increased.

Due to its beneficial properties for wound treatment, the invention is also directed to a composition as described herein for medical use. The composition is particularly advantageous for treatment of wounds in order to improve wound healing, e.g. in order to prevent microbial growth, debride the wound and/or alleviate local inflammations in and/or around the wound. Consequently the invention is also directed to a composition comprising nanoparticles of TiO₂ having a mean particle diameter (D₅₀) of about 3-150 nm at a concentration of about 1-2000 g/L and H₂O₂ at a final concentration at about 2.5-25 % by volume for medical use. Preferably for this aspect of the invention, the preferred compositions described herein are used. In particular, the mean particle diameter of the TiO₂ nanoparticles is about 20-50 nm. The term medical use includes all kinds of medical uses, such as medical veterinary use and medical use for humans.

The suitable dosage regime for treating a wound in accordance with the present invention of course depends on factors such as size, depth, width and character of the wound.

Preferably, most of the wounded area should be covered by the composition according to the invention when treated, unless the wounded area is too large, such as about > 100 cm². If the wounded area is too large it might be necessary to treat only some parts of the wound at the time, to avoid discomfort for the patient. However, in most cases, approximately 0.1 to 1.0 ml/cm² will be a suitable dosage for treating a wound with a composition according to the present invention, but this may also vary depending upon the viscosity of the composition, as well as which bandage material that is used.

The compositions of the present invention as disclosed herein may also be combined with an analgesic treatment of choice, such as for example Lidocain or Bupivacain, to alleviate pain and/or inflammation in a patient when the wound is being treated.

As mentioned above, one of the advantageous properties of the composition of the invention is that it is antimicrobial and that it may prevent microbial growth and/or kill microorganisms, preferably in and/or around a wound. By "antimicrobial" in the present context refers to the ability to repress and/or prevent the growth of a microorganism in or on a subject. The term may also refer to the killing of the microorganism. In the present context, the metal oxide, such as the titanium oxide provides anti-microbial effects by the production of free radicals, which are released after photo activation (i.e. in the present context activation with H₂O₂), which helps in fending off microbes. The active radicals attack the cell membrane of microorganism such as bacteria and fungi. Once the radicals have killed the bacteria, they return to their original form TiO₂. Examples of microorganisms are bacteria, fungi and viruses. The present invention is therefore also directed to the use of a composition as described herein for the manufacture of a pharmaceutical composition for preventing microbial growth, such as bacterial, fungal and/or viral growth and propagation. Consequently, the invention is also directed to the use of a composition comprising nanoparticles of TiO₂ having a mean particle diameter (D₅₀) of about 3-150 nm at a concentration of about 1-2000 g/L and H₂O₂ at a final concentration at about 2.5-25 % by volume for the preparation of a pharmaceutical composition for preventing microbial growth. The invention is therefore also directed to the composition of the invention for use for preventing microbial growth, such as bacterial, fungal and/or viral growth and propagation. Also, another aspect of the invention is directed to a method for preventing microbial growth in and/or around a wound comprising the step of administering a therapeutically effective amount of the composition of the invention to a subject in need thereof. Preferably also for these aspects of the invention, the preferred compositions as described herein are used. In particular, the mean particle diameter of the TiO₂ nanoparticles is about 20-50 nm.

Also, the composition of the invention may be used for wound debridement. Another aspect of the invention is therefore directed to the use of the composition of the invention for the manufacture of a pharmaceutical composition for wound debridement. Consequently, the invention is also directed to the use of a composition comprising nanoparticles of TiO₂ having a mean particle diameter (D₅₀) of about 3-150 nm at a concentration of about 1-2000 g/L and H₂O₂ at a final concentration at about 2.5-25 % by volume for the preparation of a pharmaceutical composition for debriding a wound. The invention is also directed to the composition of the invention for use for debriding a wound. Also, another aspect of the invention is directed to a method for debriding a wound comprising the step of administering a therapeutically effective amount of the composition of the invention to a subject in need thereof. Preferably also for these aspects of the invention, the preferred compositions as described herein are used. In particular, the mean particle diameter of the TiO₂ nanoparticles is about 20-50 nm.

By "wound debridement" is in the present context meant the medical removal of a patient's dead, damaged, and/or infected tissue to improve the healing potential of the remaining healthy tissue.

The composition of the invention also is able to alleviate local inflammation, i.e. the composition is an anti-inflammatory composition. The invention is therefore also directed to the use of the composition of the invention for the manufacture of a pharmaceutical composition for locally alleviating inflammation, preferably in and/or around a wound. Consequently, the invention is also directed to the use of a composition comprising nanoparticles of TiO₂ having a mean particle diameter (D₅₀) of about 3-150 nm at a concentration of about 1-2000 g/L and H₂O₂ at a final concentration at about 2.5-25 % by volume for the preparation of a pharmaceutical composition for locally alleviating inflammation. The invention is also directed to the composition of the invention for use for locally alleviating inflammation, preferably in and/or around a wound. Another aspect of the invention is directed to a method for treating and/or alleviating local inflammations in and/or around a wound comprising the step of administrating a therapeutically effective amount of the composition of the invention to a subject in need thereof. Preferably also for these aspects of the invention, the preferred compositions as described herein are used. In particular, the mean particle diameter of the TiO₂ nanoparticles is preferably about 20-50 nm.

Due to the antimicrobial and wound debriding properties of the composition of the invention, the invention is also directed to the use of the composition of the invention for the manufacture of a pharmaceutical composition for simultaneously preventing microbial growth and debriding a wound. Consequently, the invention is also directed to the use of a composition comprising nanoparticles of TiO₂ having a mean particle diameter (D₅₀) of about 3-150 nm at a concentration of about 1-2000 g/L and H₂O₂ at a final concentration at about 2.5-25 % by volume for the preparation of a pharmaceutical composition for simultaneously preventing microbial growth and debriding a wound. The invention is also directed to the composition of the invention for use for simultaneously preventing microbial growth and debriding a wound. Another aspect of the invention is directed to a method for simultaneously preventing microbial growth and debriding in and/or around a wound comprising the step of administrating the composition of the invention to a subject in need thereof. Preferably also for these aspects of the invention, the preferred compositions as described herein are used. Accordingly, in a preferred aspect the mean particle diameter of the TiO₂ nanoparticles is about 20-50 nm.

The composition of the invention may be administered to the wound, i.e. to the wound area, but it may also be administered to the area adjacent to the wound to debride and/or prevent microbial growth and/or alleviate a local inflammation around the wound. By the administration the wound, and/or the area around the wound, is cleaned and organic debris, microorganisms etc. is removed from the wound. Also, as described further below, the composition may be present in a medical or cosmetical product or device. Also, the composition of the invention may suitably be applied to a wound via a syringe.

The composition of the invention is particularly suitable for the medical uses specified herein as the composition will not just be present on the surface of the wound, but also penetrate the wound and kill bacteria deeper in the wound site due to the nanosize of the TiO₂ particles. It is well recognised amongst the scientific community that the deeper laying bacteria are the main cause for chronic wounds and it is therefore important to reach also these bacteria, something that is possible by the use of the present composition. In addition, the risks for negative side effects, such as allergy, are minimized due to the well documented biocompatible properties of TiO₂. Also, compared to e.g. treatment of wounds with silver ions, the composition of the invention does not scar or discolour the wound. In addition, no sensitization occurs to the composition of the invention, as may occur when e.g. silver is used for wound treatment, and the composition may therefore be used often and for long periods of time. Compared to antibiotics, the use of the composition of the invention for wound treatment does not cause a risk for development of resistance against it and also, compared to antibiotics, the composition comprising the activated TiO₂ nanoparticles kills and/or prevents growth of microorganisms unselectively. The composition of the invention is particularly suitable for the treatment of deep wounds as there is no need to active the TiO₂ nanoparticles by UV light. As previously mentioned, UV light would not be able to reach sufficiently deep down in a wound in order to allow activation of the TiO₂ nanoparticles and would also offer other disadvantages as mentioned herein. Therefore, the present composition can with advantage be used to treat wounds previously difficult to treat.

Due to the small size of the TiO₂ nanoparticles in the composition of the invention, the nanoparticles may be fagocytized by macrofages and thereby removed from the wound without having to be washed away after the composition is applied and have had time to act in the wound. However, the nanoparticles of TiO₂ are not so small that there is any risk for cell wall penetration. Also, when the activated nanoparticles of TiO₂ (i.e. the radicals as described above) have performed their task, e.g. killed microorganisms, they return to their less active state, i.e. unactivated TiO₂. This can e.g. be compared to silver ions that continue to be biologically active, which also means that they continue to be toxic to the environment as they are not inactivated after they have performed their tasks. The TiO₂ nanoparticles in the composition of the invention may however be reactivated to form the active radical species, e.g. by the careful application of UV-light to a wound or a wound dressing having the composition of the invention applied thereto. Without being bound by theory, it is also possible that the nanoparticles of TiO₂ may be reactivated by H₂O₂ formed naturally by the cells. Also the TiO₂ nanoparticles are anti-inflammatory even when they are not in their active state and may as such also have an anti-inflammatory effect.

Moreover, the activated nanosized TiO₂ particles in the composition of the invention actively modulate immune responses, acribate macrophages and stimulate the healing process. This means that such particles will not just kill microorganisms in the wound, but also stimulate the wound in the healing process as well.

In the present context, a "wound" is a type of injury in which skin e.g. is torn, cut or punctured (an open wound), or where blunt force trauma causes a contusion (a closed wound). In pathology, it specifically refers to a sharp injury which damages the dermis of the skin. A wound is often subject to microbial infection. The composition of the invention is particularly suitable for patients with wounds having increasing signs of bacterial influence, redness, odour, pain and/or exudates. Also the compositions are particularly useful when signs of pseudomonas and oedema are shown, when wound healing does not progress normally, or when the skin temperature around the wound is increased.

Examples of wound types which particularly benefit from the composition of the invention are skin cancer wounds, chronic wounds due to bacterial colonization and diabetic ulcers. Also, surgical wounds, such as microsurgical wounds, are particularly subject to bacterial infection and benefit from being cleaned with the composition of the invention in order to debride the wound, alleviate local inflammation and/or prevent microbial growth.

The composition of the invention is preferably added to a medical product or device and/or cosmetic product or device. The present invention is therefore in another aspect directed to a medical product or device and/or cosmetic product or device comprising a composition as disclosed herein.

In the following examples of medical and/or cosmetic products and devices comprising the composition of the invention, depending on the use of the product/device, it may be denoted as a medical or a cosmetic product/device.

The composition of the invention is preferably added to a wet wound dressing, a so called moist wound care (MWC) product. Such wound dressings include
- Hydrocolloid wound dressings- Refers to a waterproof, occlusive dressing which consists of a mixture of pectins, gelatins, sodium carboxymethylcellulose, and finally elastomers. This dressing ensures an environment which promotes autolysis to debride wounds which are sloughy or necrotic.
- Hydrofibre wound dressings - Wound dressing which is highly absorbent made up of complete hydrocolloid. Here, the hydrocolloid is spun into fibers and needled to create a soft, nonwoven, fleece-like dressing in the form of sheet or ribbon. Anti-bacterial effect enhanced by silver. Hydrofibre appears to be an alternative to alginate dressing.
- Hydrogels wound dressings - Dressings in the form of sheet or gel which are used for shallow or low-exuding wounds. This hydrogel is ideal for cavities and are effective for desloughing and debriding wounds.
- Foam wound dressings - Name given to a dressing designated from polyurethane which is non-adherent and can absorb large amounts of exudates in addition to being utilized as secondary dressings. This foam dressing is available impregnated with charcoal along with a waterproof backing.
- Transparent wound dressings - Dressings that are flexible with limited absorption, generally used as secondary dressing.

Another type of wound dressing to which the composition of the invention preferably is added to is a wound dressing consisting of one or several coated solid(s), pliant material(s), said coating(s) comprising a homogenous and substantially amorphous metal oxide layer comprising predominantly titanium oxide (i.e. 50% or more titanium oxide) and having a thickness of 200 nm or less, preferably 100 nm or less. The metal oxide layer may additionally comprise one or more compounds selected from the group consisting of N, C, S, Cl, and/or one or more compounds selected from the group consisting of Cl and N, and/or one or more compounds selected from the group consisting of Ag, Au, Pd, Pt, Fe, Cl, F, Pb, Zn, Zr, B, Br, Si, Cr, Hg, Sr, Cu, I, Sn, Ta, V, W, Co, Mg, Mn and Cd or oxides of any of the metals and/or one or more compounds selected from the group consisting of SnO₂, CaSnO₃, FeGaO₃, BaZrO₃, ZnO, Nb₂O₅, CdS, ZnO₂, SrBi₂O₅, BiAlVO₇, ZnlnS₄, K₆Nb_{10.8030}, and/or a combination of compounds selected from said groups of compounds, wherein said one or more compound(s) selected from one or more group(s) of compounds are dispersed substantially homogenous within said metal oxide layer. The solid, pliant material forming part of the wound dressing is here preferably selected from the group consisting of polyurethane (PUR, TPU, PCU), polyamid, (PA), polyether, polyethylene, (PE), polyester, polypropylene, (PP), poly(tetrafluoroethylene) (PTFE), silicones, cellulose, silk and cotton. Such a wound dressing may also comprise an inner linkage layer between the solid, pliant material and the metal oxide layer. Preferably, said linkage layer comprises one or more of the compound(s) Al₂O₃, N or an oxide thereof, S or an oxide thereof, Al₂O₃, SiO₂, Ta₂O₅, ZrO₂, HfO₂, ZnO, MgO, Cr₂O₃, Co₂O₃, NiO, FeO, Ga₂O₃, GeO₂, V₂O₅, Y₂O₃, rare earth oxides, CaO, In₂O₃, SnO₂, PbO, MoO₃ and WO₃, TiN, TaN, SiN₄, AIN, Hf₃N₄ and Zr₃N₄, or any combination thereof. The addition of a linkage layer to the solid, pliant material provides the advantage that the metal oxide is more firmly attached to said pliant material, and that it provides protection against damaging UV light exposure and against potentially harmful reactive oxygen species produced during photoactivation of the layer. Also, the presence of the linkage layer provides improved properties to the solid pliant material as it makes the photocatalytic layer adhere even stronger to the material. The material can hence be re-activated by photo activation several times without being removed from the wound, thus relieving the patient from discomfort and pain due to frequent dressing changes, and at the same time leaving the wound to heal up undisturbed. Such a wound dressing is preferably manufactured by the use of ALD technology (Atomic Layer Deposition). ALD is a self-limiting, sequential surface chemistry method that deposits conformal thin-films of materials onto substrates of varying compositions. ALD film growth is self-limited and based on surface reactions, which makes achieving atomic scale deposition control possible. By keeping the precursors separate throughout the coating process, atomic layer control of film grown can be obtained as fine as ~ 0.1 angstroms per monolayer. ALD grown films are conformal, pin-hole free, and chemically bonded to the substrate. With ALD it is possible to deposit coatings perfectly uniform in thickness inside deep trenches, porous media and around particles. The film thickness range provided by the ALD technology is usually 1-500 nm. When applying ALD technology on a solid pliant material, a substantially lower temperature is used, typically in the range of lower than 300°C.

The composition of the invention is preferably added to wound dressings to aid in debriding the wound and/or prevent microbial infection and/or inflammation thereof. Also, as the TiO₂ nanoparticles may be re-activated by the exposure to UV-light, such reactivation means that the dressings do not have to be changed as often as is normally necessary, which increases patient comfort and leaves the wound more undisturbed for healing. A wet wound dressing may also comprise the TiO₂ nanoparticles on it and immediately before its use on a wound, H₂O₂ be added to the wound dressing in the correct amount and concentration to activate the TiO₂ nanoparticles.

The composition of the invention may also be added to commonly available wound debridement products.

Also, the composition of the invention is preferably added to a medical product such as a crème or ointment. Equally well, the composition may be added to a shampoo, e.g. for use of subjects affected with psoriasis or dandruff. A deodorant having the composition results in a product for prevention of body odour.

The are numerous other examples of products or devices that the composition of the invention is suitable to be added to, such as liniments, enema, crèmes and wash solutions for acne treatment and prevention, in order to debride wounds, prevent microbial growth and/or alleviate inflammations.

The composition of the invention may preferably be formulated together with one or more pharmaceutically or cosmetically acceptable excipient(s), carrier(s) and/or adjuvant(s).

Products or devices comprising the composition of the invention may be in form of, e.g., solid, semi-solid or fluid compositions such as bioabsorbable patches, drenches, dressings, hydrogel dressings, hydrocolloid dressings, films, foams, sheets, bandages, plasters, delivery devices, implants,
sprays, aerosols, inhalation devices,
gels, hydrogels, pastes, ointments, creams, soaps, suppositories, vagitories,
solutions, dispersions, suspensions, emulsions, mixtures, lotions, shampoos, enemas,
and in other suitable forms such as, e.g., implants or coating of implants or in a form suitable for use in connection with implantation or transplantation.

Products for application to the skin or to the mucosa are considered most important in connection with the present invention. Thus, a product comprising the composition of the invention may be adapted for administration by any suitable route, for example by topical (dermal), buccal, nasal, aural, rectal or vaginal administration. Furthermore, the composition of the invention may be adapted to administration in connection with surgery, e.g. in connection with incision within the body in order to promote healing of internal wounds and soft tissue damages.

Products and devices for medical/cosmetic use comprising the composition of the invention may be formulated according to conventional pharmaceutical practice, see, e.g., "Remington's Pharmaceutical Sciences" and "Encyclopedia of Pharmaceutical Technology", edited by Swarbrick, J. & J. C. Boylan, Marcel Dekker, Inc., New York, 1988.

A pharmaceutically or cosmetically acceptable excipient is a substance which is substantially harmless to the individual to which the composition is to be administered. Such an excipient normally fulfils the requirements given by the national health authorities. Official pharmacopoeias such as e.g. the British Pharmacopoeia, the United States of America Pharmacopoeia and The European Pharmacopoeia set standards for pharmaceutically acceptable excipients.

The pharmaceutically acceptable excipients may include solvents, buffering agents, preservatives, humectants, chelating agents, antioxidants, stabilizers, emulsifying agents, suspending agents, gel-forming agents, ointment bases, penetration enhancers, perfumes, and skin protective agents.

Examples of solvents are e.g. water, alcohols, vegetable or marine oils (e.g. edible oils like almond oil, castor oil, cacao butter, coconut oil, corn oil, cottonseed oil, linseed oil, olive oil, palm oil, peanut oil, poppyseed oil, rapeseed oil, sesame oil, soybean oil, sunflower oil, and teaseed oil), mineral oils, fatty oils, liquid paraffin, polyethylene glycols, propylene glycols, glycerol, liquid polyalkylsiloxanes, and mixtures thereof.

Examples of buffering agents are e.g. citric acid, acetic acid, tartaric acid, lactic acid, hydrogenphosphoric acid, diethylamine etc.

Suitable examples of preservatives for use in the compositions are parabens, such as methyl, ethyl, propyl p-hydroxybenzoate, butylparaben, isobutylparaben, isopropylparaben, potassium sorbate, sorbic acid, benzoic acid, methyl benzoate, phenoxyethanol, bronopol, bronidox, MDM hydantoin, iodopropynyl butylcarbamate, EDTA, benzalconium chloride, and benzylalcohol, or mixtures of preservatives.

Examples of humectants are glycerin, propylene glycol, sorbitol, lactic acid, urea, and mixtures thereof.

Examples of chelating agents are sodium EDTA and citric acid.

Examples of antioxidants are butylated hydroxy anisole (BHA), ascor¬bic acid and derivatives thereof, tocopherol and derivatives thereof, cysteine, and mixtures thereof.

Examples of emulsifying agents are naturally occurring gums, e.g. gum acacia or gum tragacanth; naturally occurring phosphatides, e.g. soybean lecithin; sorbitan monooleate derivatives; wool fats; wool alcohols; sorbitan esters; monoglycerides; fatty alcohols;, fatty acid esters (e.g. triglycerides of fatty acids); and mixtures thereof.

Examples of suspending agents are e.g. celluloses and cellulose derivatives such as, e.g., carboxymethyl cellulose, hydroxyethylcellulose, hydroxypropylcellulose, hydroxypropylmethylcellulose, carraghenan, acacia gum, arabic gum, tragacanth, and mixtures thereof.

Examples of gel bases, viscosity-increasing agents or components which are able to take up exudate from a wound are: liquid paraffin, polyethylene, fatty oils, colloidal silica or aluminium, zinc soaps, glycerol, propylene glycol, tragacanth, carboxyvinyl polymers, magnesium-aluminium silicates, Carbopol®, hydrophilic polymers such as, e.g. starch or cellulose derivatives such as, e.g., carboxymethylcellulose, hydroxyethylcellulose and other cellulose derivatives, water-swellable hydrocolloids, carragenans, hyaluronates (e.g. hyaluronate gel optionally containing sodium chloride), and alginates including propylene glycol aginate.

Examples of ointment bases are e.g. beeswax, paraffin, cetanol, cetyl palmitate, vegetable oils, sorbitan esters of fatty acids (Span), polyethylene glycols, and condensation products between sorbitan esters of fatty acids and ethylene oxide, e.g. polyoxyethylene sorbitan monooleate (Tween).

Examples of hydrophobic or water-emulsifying ointment bases are paraffins, vegetable oils, animal fats, synthetic glycerides, waxes, lanolin, and liquid polyalkylsiloxanes.

Examples of hydrophilic ointment bases are solid macrogols (polyethylene glycols).

Other examples of ointment bases are triethanolamine soaps, sulphated fatty alcohol and polysorbates.

Examples of other excipients are polymers such as carmelose, sodium carmelose, hydroxypropylmethylcellulose, hydroxyethylcellulose, hydroxypropylcellulose, pectin, xanthan gum, locust bean gum, acacia gum, gelatin, carbomer, emulsifiers like vitamin E, glyceryl stearates, cetanyl glucoside, collagen, carrageenan, hyaluronates and alginates.

Suitable dispersing or wetting agents are, for example, naturally occurring phosphatides, e.g., lecithin, or soybean lecithin; condensation products of ethylene oxide with e.g. a fatty acid, a long chain aliphatic alcohol, or a partial ester derived from fatty acids and a hexitol or a hexitol anhydride, for example polyoxyethylene stearate, polyoxyethylene sorbitol monooleate, polyoxyethylene sorbitan mono¬oleate, etc.

Suitable suspending agents are, e.g., naturally occurring gums such as, e.g., gum acacia, xanthan gum, or gum tragacanth; celluloses such as, e.g., sodium carboxymethylcellulose, microcrystalline cellulose (e.g. Avicel® RC 591, methylcellulose); alginates such as, e.g., sodium alginate, etc.

For application to the rectal or vaginal mucosa, suitable compositions according to the invention include suppositories (emulsion or suspension type), enemas, and rectal gelatin capsules (solutions or suspensions). Appropriate pharmaceutically acceptable suppository bases include cocoa butter, esterified fatty acids, glycerinated gelatin, and various watersoluble or dispersible bases like polyethylene glycols and polyoxyethylene sorbitan fatty acid esters. Various additives like, e.g., enhancers or surfactants may be incorporated.

For application to the nasal mucosa (as well as to the oral mucosa), sprays and aerosols for inhalation are suitable.

In another aspect the invention is directed to a kit comprising a first container comprising TiO₂ having a mean particle diameter (D₅₀) of about 3-150 nm and a second container comprising H₂O₂, which, when mixed with each other, provide a composition according to the herein exemplified and/or preferred compositions. The TiO₂ nanoparticles are preferably present in an aqueous solution. By the mixing of the contents of the two containers, the TiO₂ particles are activated and the active radical nanoparticles are formed. A kit according to the present invention may also comprise one or more device(s), such as a syringe, for administration of the composition. Also, the kit may contain instructions for mixing and/or administration of the composition to a subject. Preferably the mean particle diameter (D₅₀) of the TiO₂ nanoparticles in such is kit is 20-50 nm.

The present invention is also directed to a method for preparing a composition as described herein, comprising the steps of mixing said nanoparticles of TiO₂ with said H₂O₂.

The present invention will now be illustrated by the below examples, which are not to be seen as limiting for the scope of the present invention.

### Experimental section

Surprisingly, the titanium dioxide nanoparticles in suspension in hydrogen peroxide (H₂O₂) were activated by H₂O₂. A synergical effect was highlighted between TiO₂ nanoparticles and H₂O₂. No UV-irradiation was necessary to catalyse the reaction.
The created suspension was tested against several organic materials. Once activated, the particles are exposing free radicals that are believed to be the reason for the anti-fouling and anti-microbial properties of the suspension.

### EXAMPLE 1 Methylene blue degradation when mixed with H₂O₂ at 15 vol.% and TiO₂ at various concentrations

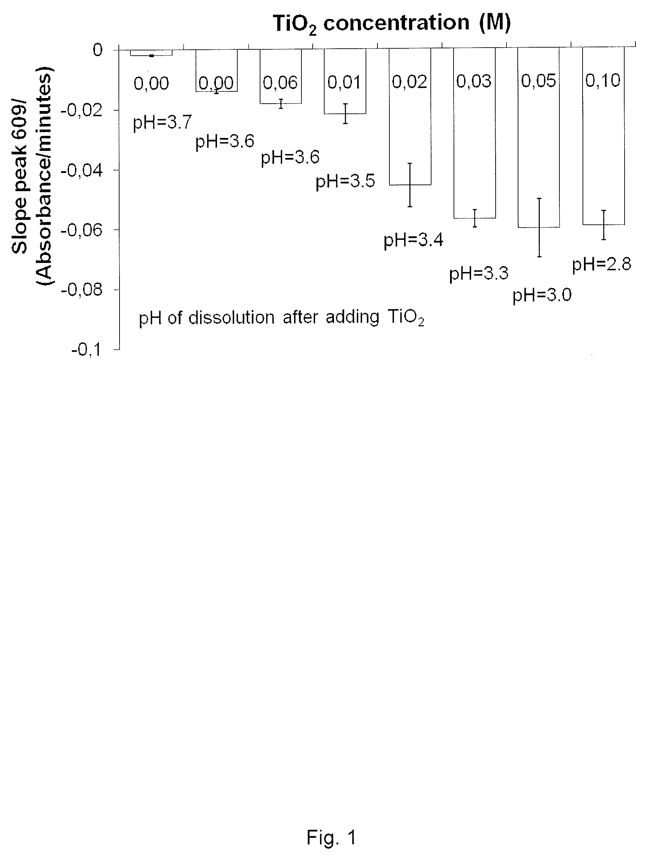

Methylene blue (MB) is a heterocyclic aromatic chemical compound with molecular formula: C₁₆H₁₈N₃SCl (AldrichSigmaAldrich, Oslo, Norway). It has many uses in a range of different fields, such as biology and chemistry. MB exemplifies organic materials and can thus be used as agent to simulate bacteria as seen in several publications and is commonly used when investigating the degradative properties of TiO₂. A degradadation of MB therefore can be used as a model for the *in vivo* degradation of organic material, such as bacteria, or dead, damaged, and/or infected tissue. At room temperature it appears as a solid, odourless, dark green powder that yields a blue solution when dissolved in water. When degradated in solution, methylene blue becomes colourless. When methylene blue is analysed by UV-vis spectrophotometry (Lambda 25, Perkin Elmer, USA), it absorbs light at 690 cm. This machine was used to quantify the degradation of MB.
The aim was to measure if methylene blue could be degradated by H₂O₂ (PERDROGEN® 30% H₂O₂ (w/w), Sigma Aldrich AS, Oslo, Norway) at 15% only, without TiO₂ or UV irradiation. Then, an increasing concentration of TiO₂ (Aeroxide P25, Evonik AG, Essen, Germany) was added to the solution until a maximum concentration of 8 g/L. The measurements were taken every 3 minutes for one hour. The pH of the suspension containing MB was also registered. The suspension was stirred prior to each measurement to prevent the deposition of the TiO₂ particles. The results are presented in figure 1.
Almost no degradation was found when only H₂O₂ (PERDROGEN® 30% H₂O₂ (w/w), Sigma Aldrich AS, Oslo, Norway) at 15vol.% was mixed with MB. Surprisingly, the presence of TiO₂ at 0.25 g/L in the suspension made the MB degradation possible. Increasing the concentration of TiO₂ nanoparticles in the suspension increased the MB degradation. Increasing the concentration of TiO₂ particles decreased slightly the pH from 3.7 without TiO₂, to 2.8 with TiO₂ at 1M.
The H₂O₂ alone could not break down the MB molecules; surprisingly TiO₂ must be present to reach this purpose.

### EXAMPLE 2 Methylene blue degradation when mixed with H₂O₂ at 15% and TiO₂ at various concentrations

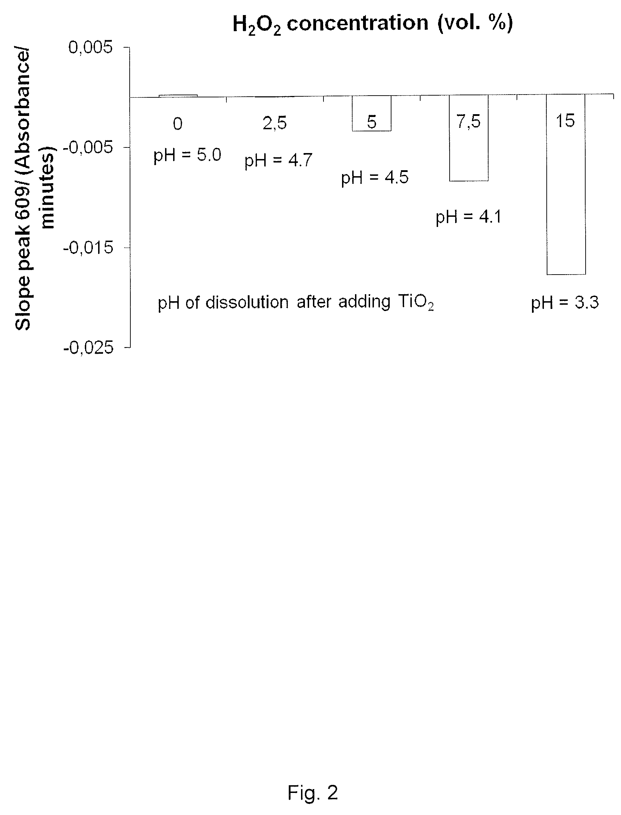

The aim was to measure if methylene blue could be degradated by TiO₂ nanoparticles only (Aeroxide P25, Evonik AG, Essen, Germany), without H₂O₂ or UV irradiation. Then, an increasing concentration of H₂O₂ (PERDROGEN® 30% H₂O₂ (w/w), Sigma Aldrich AS, Oslo, Norway) was added to the suspension until a maximum concentration of 15 vol.%. The procedure of the experiment was the same as explained in example 1.
The results are presented in figure 2. Almost no degradation was found when only TiO₂ at 0.5 g/L was mixed with MB. Adding 5 vol.% concentrated H₂O₂ increased the MB degradation. Increasing the concentration of H₂O₂ in the suspension increased the MB degradation in a linear way. Increasing the concentration of H₂O₂ decreased the pH from 4.9 without H₂O₂, to 3.3 with H₂O₂ at 15vol.%.
The TiO₂ alone could not break down the MB molecules; H₂O₂ must be present to reach this purpose.

### EXAMPLE 3 Methylene blue degradation when mixed with the suspension of H₂O₂ at 5% and TiO₂ at 1.6 g/L doped with NaCl at 0.8 g/L or NaF at 0.4 g/L

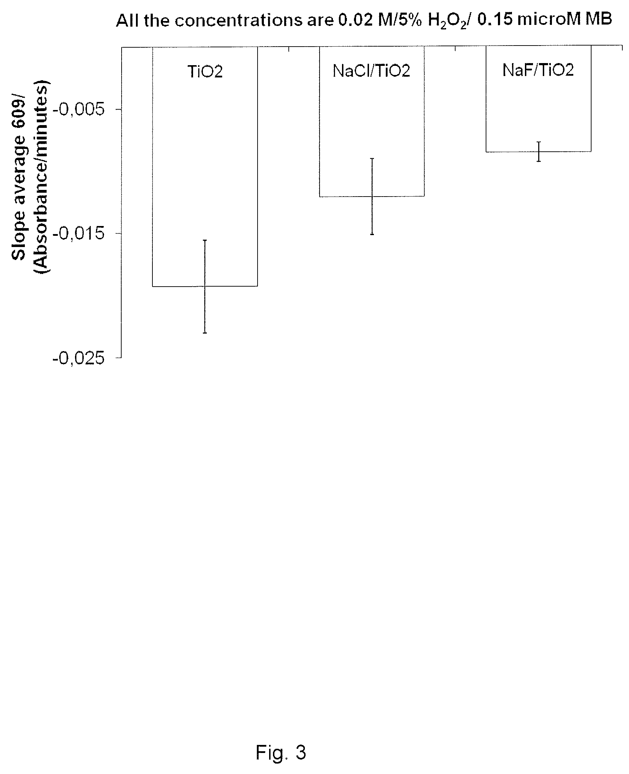

From examples 1 and 2, H₂O₂ (PERDROGEN® 30% H₂O₂ (w/w), Sigma Aldrich AS, Oslo, Norway) and TiO₂ (Aeroxide P25, Evonik AG, Essen, Germany) concentrations were chosen in order to create a suspension that uses this synergical effect but as well which could be used in a physiological environment ([H₂O₂]<6vol.% and pH>3).
The aim was to discover whether or not NaCl and NaF salts could further increase the MB degradation when introduced in the chosen suspension.
The results are presented in figure 3.
Interestingly, doping the suspension with NaCl reduced the MB degradation by half compared with the suspension with only TiO₂ and H₂O₂. Doping with NaF (Sigma Aldrich AS, Oslo, Norway) was even stronger in reducing the MB degradation, almost preventing this degradation to occur compared with the suspension with only TiO₂ and H₂O₂.
Doping the H₂O₂/TiO₂ suspension with NaF (Sigma Aldrich AS, Oslo, Norway) and NaCl (Sigma Aldrich AS, Oslo, Norway) salts did not increase the MB degradation.

### EXAMPLE 4 pH measurements of various suspensions

The pH in wound healing is an important factor, and the suspension's pH can be altered by different TiO₂ (Aeroxide P25, Evonik AG, Essen, Germany) concentrations and other added components.. A laboratory pH meter (pH Meter Lab 850 Set with Blueline 14pH Electrode, Scott Glass Ltd, Stafford, UK) was used to measure the pH in different solutions. Below is a list of the resulting pH after the given concentrations:
1. A mixture of 5 vol.% H₂O₂ and 1.6 g/L1.6 g/L TiO₂ resulted in a pH = 4.4 ± 0.1.
2. A mixture of 5 vol.% H₂O₂ + 1.6 g/L NaCl resulted in a pH = 5.2 ± 0.1
3. A mixture of 5 vol.% H₂O₂ 2 + 1.6 g/L NaF resulted in a pH = 6.1 ± 0.0
4. A mixture of 5 vol.% H₂O₂ + 1.6 g/L NaCl + 1.6 g/L TiO₂ resulted in a pH = A 4.2 ± 0.1
5. A mixture of 5% H₂O₂ + 1.6 g/L NaF + 1.6 g/L Ti02 resulted in a pH = 5.9 ± 0.0 The range of the various tested suspension went from 4.2 up to 6.1.

### EXAMPLE 5 Suspension with higher viscosity

The solution of 2 g/L of TiO₂ (Aeroxide P25, Evonik AG, Essen, Germany) and 5 vol. % H₂O₂ (PERDROGEN® 30% H₂O₂ (w/w), Sigma Aldrich AS, Oslo, Norway) from example 1 and 2, where modified in order to achieve higher viscosity by adding higher quantitives of (Aeroxide P25, Evonik AG, Essen, Germany). At 1000 g/L the suspension became a thick slurry.

### EXAMPLE 6 ANTI-BACTERIAL EFFECT OF ACTIVATED TIO₂ NANOPARTICLES ON STAPHYLOCOCCUS AUREUS

This example describes the anti-bacterial potential of the activated TiO₂ nanoparticles.

### Bacteria:

Staphylococcus aureus (S. aureus) are important human commensal and opportunistic pathogens responsible for a wide range of infections. They are one of the most known bacteria responsible for post-surgery infection. Therefore, S. aureus will be chosen for this experiment.

### Procedure:

Small squares (5x5mm²) of wound dressing will be cut.

The anti-bacterial effect of the three wound dressings will be treated with a suspension of H₂O₂ (PERDROGEN® 30% H₂O₂ (w/w), Sigma Aldritch AS, Oslo, Norway) at 3, 5 and 25 vol.% that will be mixed with TiO₂ (Aeroxide P25, Evonik AG, Essen, Germany) at 0.5, 1.6 and 20 g/L. The control will be wound dressing without any suspension and wound dressing with treated only H₂O₂ (PERDROGEN® 30% H₂O₂ (w/w), Sigma Aldritch AS, Oslo, Norway) at 5 vol.%.
These wound dresssing will prior to the mixing of suspension loaded with 500µl from a broth of S. aureus will be diluted in 4ml of PBS (Dulbecco's PBS, Sigma-Aldrich, St Louis, MO, USA) (stock solution). A drop of 10µl of this stock solution will be placed on the top of the wound dressing. Once the UV light exposure of the test groups reached, the small squares of wound dressing will be individually placed in 1.5 ml Eppendorf tubes containing 500µl of cell culture medium (without antibiotics) of from Invitrogen (GIBSCO MEM, Invitrogen, Carlsbad, CA, USA). All the Eppendorf tubes containing the wound dressing and the bacteria will be placed in an incubator, in the dark, at 37°C for 20 hours. After 20 hours, all the samples will be taken out of the incubator. A Spectrometer (Perkin Elmer UV-Vis 200, Oslo, Norway) will be calibrated with only 700µl of cell media for the base line. Then, the three Eppendorf tubes containing only 500µl of cell media + 10µl of the stock solution will be analysed. Then, one by one the test tubes will be shaked and a volume of 400µl from each tube will be mixed with 300µl of cell media. The 1.5ml cuvettes contained 700µl of liquid to be analysed.

### EXAMPLE 7 ANTI-BACTERIAL EFFECT OF ACTIVATED TIO2 NANOPARTICLES ON PSEUDOMONAS AEROGINOSA BACTERIA

This example describes the anti-bacterial potential of the activated TiO₂ nanoparticles.

### Bacteria:

Pseudomonas aeroginosa bacteria are opportunistic pathogens responsible for a wide range of infections, and often found in chronical wounds.

### Procedure:

Small squares (5x5mm²) of wound dressing will be cut.

The anti-bacterial effect of the three wound dressings will be treated with a suspension of H₂O₂ (PERDROGEN® 30% H₂O₂ (w/w), Sigma Aldritch AS, Oslo, Norway) at 3, 5 and 25 that will be mixed with TiO₂ (Aeroxide P25, Evonik AG, Essen, Germany) at 0.5, 1.6 and 20 g/L. The control will be wound dressing without any suspension and wound dressing with treated only H₂O₂ (PERDROGEN® 30% H₂O₂ (w/w), Sigma Aldritch AS, Oslo, Norway) at 5 vol.%.

These wound dresssing will prior to the mixing of suspension loaded with 500µl from a broth of S. aureus will be diluted in 4ml of PBS (Dulbecco's PBS, Sigma-Aldrich, St Louis, MO, USA) (stock solution). A drop of 10µl of this stock solution will be placed on the top of the wound dressing. Once the UV light exposure of the test groups reached, the small squares of wound dressing will be individually placed in 1.5 ml Eppendorf tubes containing 500µl of cell culture medium (without antibiotics) of from Invitrogen (GIBSCO MEM, Invitrogen, Carlsbad, CA, USA). All the Eppendorf tubes containing the wound dressing and the bacteria will be placed in an incubator, in the dark, at 37°C for 20 hours. After 20 hours, all the samples will be taken out of the incubator. A Spectrometer (Perkin Elmer UV-Vis 200, Oslo, Norway) will be calibrated with only 700µl of cell media for the base line. Then, the three Eppendorf tubes containing only 500µl of cell media + 10µl of the stock solution will be analysed. Then, one by one the test tubes will be shaked and a volume of 400µl from each tube will be mixed with 300µl of cell media. The 1.5ml cuvettes contained 700µl of liquid to be analysed.

### EXAMPLE 8 ANTI-BACTERIAL EFFECT OF ACTIVATED TIO₂ NANOPARTICLES ON E.COLI BACTERIA

This example describes the anti-bacterial potential of the activated TiO₂ nanoparticles.

### Bacteria:

E.coli bacteria are sometime present in chronical wounds.

### Procedure:

Small squares (5x5mm²) of wound dressing will be cut.
The anti-bacterial effect of the three wound dressings will be tested with a suspension of H₂O₂ (PERDROGEN® 30% H₂O₂ (w/w), Sigma Aldritch AS, Oslo, Norway) at 3,5 and 7.5 that will be mixed with TiO₂ (Aeroxide P25, Evonik AG, Essen, Germany) at 0.5, 1.6 and 20 g/L. The control will be wound dressing without any suspension and wound dressing with treated only H₂O₂ (PERDROGEN® 30% H₂O₂ (w/w), Sigma Aldritch AS, Oslo, Norway) at 5 vol.%.
These wound dressings will prior to the mixing of suspension loaded with 500µl from a broth of S. aureus will be diluted in 4ml of PBS (Dulbecco's PBS, Sigma-Aldrich, St Louis, MO, USA) (stock solution). A drop of 10µl of this stock solution will be placed on the top of the wound dressing. Once the UV light exposure of the test groups reached, the small squares of wound dressing will be individually placed in 1.5 ml Eppendorf tubes containing 500µl of cell culture medium (without antibiotics) of from Invitrogen (GIBSCO MEM, Invitrogen, Carlsbad, CA, USA). All the Eppendorf tubes containing the wound dressing and the bacteria will be placed in an incubator, in the dark, at 37°C for 20 hours. After 20 hours, all the samples will be taken out of the incubator. A Spectrometer (Perkin Elmer UV-Vis 200, Oslo, Norway) will be calibrated with only 700µl of cell media for the base line. Then, the three Eppendorf tubes containing only 500µl of cell media + 10µl of the stock solution will be analysed. Then, one by one the test tubes will be shaked and a volume of 400µl from each tube will be mixed with 300µl of cell media. The 1.5ml cuvettes contained 700µl of liquid to be analysed.

### EXAMPLE 9 ANTI-BACTERIAL EFFECT OF ACTIVATED TIO₂ NANOPARTICLES DOPED WITH FLUORINE ON STAPHYLOCOCCUS AUREUS

This example describes the anti-bacterial potential of the activated TiO₂ nanoparticles.

### Bacteria:

Staphylococcus aureus (S. aureus) are important human commensal and opportunistic pathogens responsible for a wide range of infections. They are one of the most known bacteria responsible for post-surgery infection. Therefore, S. aureus will be chosen for this experiment.

### Procedure:

Small squares (5x5mm²) of wound dressing will be cut.
The anti-bacterial effect of the three wound dressings will be treated with a suspension of H₂O₂ (PERDROGEN® 30% H₂O₂ (w/w), Sigma Aldrich AS, Oslo, Norway) at 5 vol.% that will be mixed with TiO₂ (Aeroxide P25, Evonik AG, Essen, Germany) at 1.6 g/L. In addition, this supension will be doped with fluorine with 0.01, 0.5, 1 and 3 atomic weight% fluorine in the surface layer. The doping will be done by added the equivalent amount of NaF into the supension. The amount of fluorine on the surface will be detected and quantified wit X-ray Photospectrometry (XPS). The control will be wound dressing without any suspension and wound dressing with treated only H₂O₂ (PERDROGEN® 30% H₂O₂ (w/w), Sigma Aldritch AS, Oslo, Norway) at 5 vol.%.
These wound dressings will prior to the mixing of suspension loaded with 500µl from a broth of S. aureus will be diluted in 4ml of PBS (Dulbecco's PBS, Sigma-Aldrich, St Louis, MO, USA) (stock solution). A drop of 10µl of this stock solution will be placed on the top of the wound dressing. Once the UV light exposure of the test groups reached, the small squares of wound dressing will be individually placed in 1.5 ml Eppendorf tubes containing 500µl of cell culture medium (without antibiotics) of from Invitrogen (GIBSCO MEM, Invitrogen, Carlsbad, CA, USA). All the Eppendorf tubes containing the wound dressing and the bacteria will be placed in an incubator, in the dark, at 37°C for 20 hours. After 20 hours, all the samples will be taken out of the incubator. A Spectrometer (Perkin Elmer UV-Vis 200, Oslo, Norway) will be calibrated with only 700µl of cell media for the base line. Then, the three Eppendorf tubes containing only 500µl of cell media + 10µl of the stock solution will be analysed. Then, one by one the test tubes will be shaked and a volume of 400µl from each tube will be mixed with 300µl of cell media. The 1.5ml cuvettes contained 700µl of liquid to be analysed.

### EXAMPLE 10 REMOVAL OF BIOFILM

This example describes the anti-bacterial potential of the activated TiO₂ nanoparticles.

### In vitro testing: biomass assessment after cleaning

### Samples preparation

Chemically pure (cp) titanium disks with a diameter of 6.2 mm and a height of 2 mm were used. They had a similar machined surface topography (turned with ripple).
After production, the disks were washed with NaOH at 40 vol.% and HNO₃ at 50 vol.% in an ultrasonic bath to remove contaminants, then were washed with deionised water to reach a neutral pH, and stored at room temperature in 70 vol.% ethanol. Thereafter, the coins were placed in eppendorf tube and steam autoclaved for sterilization.
Four chemical decontamination agents were selected for the in vitro testing: sterile saline H₂O (VWR, Oslo, Norway), Chlorhexidine, 3 vol.% H₂O₂ (VWR, Oslo, Norway), a mixture of 3 vol.% H₂O₂ and 2 g/L TiO₂ (2g nano particles: P25 Aeroxide, Degussa Evonik, Evonik Industries AG, Essen, Germany).

### Inoculation, cleaning and analysis

15 sterile titanium disks per groups were inoculated. The control group was inoculated with brain heart infusion broth (BHI) only, while the test groups (four) were inoculated with the bacteria culture (10 µl Staphylococcus epidermidis + 5 ml BHI). The incubation time was set at 24 h at 35°C in an aerobic atmosphere. The discs were then be transferred to new wells, rinsed with sterile saline water, then exposed to the four selected chemical agents for two minutes, then rinsed again with sterile saline water. The amount of biofilm present on the surface of the titanium samples was assessed by using the safranin staining method: 10 min exposure to a 0.1% solution of safranin, then rinsed with distilled water, air dried, and exposed to a solution of 30% acetic acid to release the colored biomass from the titanium surfaces. The intensity of the staining was analyzed using a Synergy HT Multi-Detection Microplate Reader (Biotek, VT, USA) with a wavelength of 530 nm.

### Results

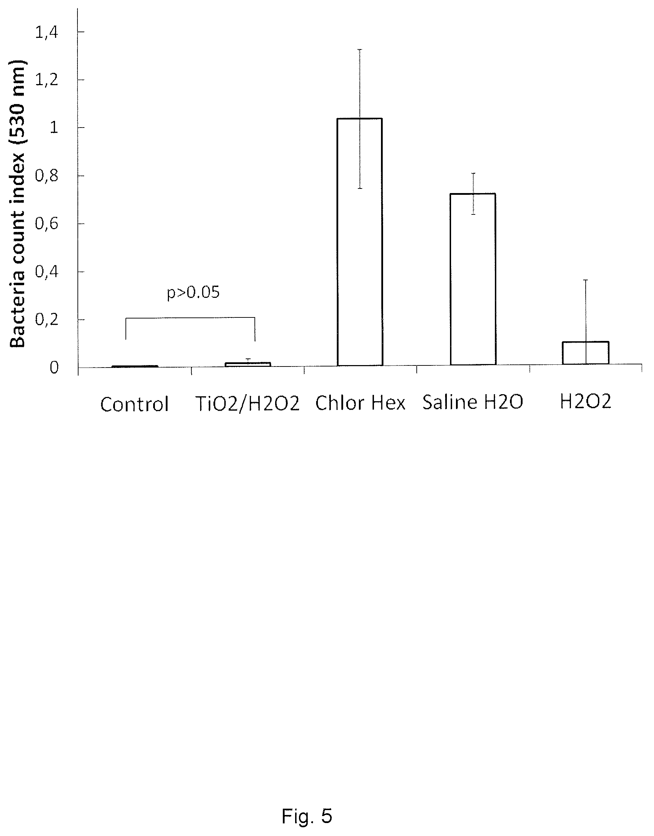

Optical density analysis in the Synergy HT Multi Detection microplate Reader revealed that the samples exposed the mixture of 3 vol. % H₂O₂ and 2 g/L TiO₂ were not significantly different than the control. Moreover, this group of samples showed a significantly lower biomass after cleaning that a single solution of 3 % H₂O₂ (Figure 5).

### EXAMPLE 11 RE-GROWTH OF BIOFILM AFTER EXPOSURE TO ACTIVATED TIO₂ NANOPARTICLES

This example describes the anti-bacterial potential of the activated TiO₂ nanoparticles in preventing biofilm re-growth after disinfection.

### In vitro testing: bacteria re-growth

Another test will be conducted in order to determine the viability of the biofilm after the disinfection. The method will be similar than for the safranin staining analysis conducted in example 10 above, from the inoculation to the disinfection using the same four products. But this time, after the disinfection step, the samples will be rinsed in NaCl and re-incubated at 35°C for four hours in pure BHI medium. The medium will be collected and analyzed using the same spectrophotometer than for the safranin staining but this time at a wavelength of 600 nm. The intensity of the absorbance will be compared between control and test groups.

The results from this experiment should show a significantly lower level of bacteria re-growth on the samples exposed to the mixture of 3 vol.% H₂O₂ and 2 g/L TiO₂ compared to the control group and to the samples exposed to H₂O₂ alone.

### References

[1] Byrne JA, Eggins BR, Brown NMD, McKinney B, Rouse M 1998 Immobilisation of Ti02 powder for the treatment of polluted water Applied Catalysis B-Environmental 17 25-36
[2] Hur JS, Koh YJ 2002 Bactericidal activity and water purification of immobilized TiO2 photocatalyst in bean sprout cultivation Biotechnology Letters 24 23-25
[3] Maness PC, Smolinski S, Blake DM, Huang Z, Wolfrum EJ, Jacoby WA 1999 Bactericidal activity of photocatalytic Ti02 reaction: Toward an understanding of its killing mechanism Applied and Environmental Microbiology 65 4094-98
[4] Yu JC, Yu JG, Ho WK, Zhang LZ 2001 Preparation of highly photocatalytic active nano-sized TiO2 particles via ultrasonic irradiation Chemical Communications 1942-43
[5] Frost&Sullivan. Global Advanced Wound Management Markets. In 2005.
[6] Frost&Sullivan. European Markets for Advanced Wound Management. In 2006.
[7] Frost&Sullivan. Western European Advanced Wound Management In 2009.
[8] Hoffmann MR, Martin ST, Choi W, Bahnemann DW 1995 Environmental Applications of Semiconductor Photocatalysis Chem. Rev 95 69-96
[9] Ollis DF 1985 Contaminant degradation in water Environ. Sci. Technol. 19 480-84
[10] Fox MA, Dulay MT 1993 Heterogeneous photocatalysis Heterogeneous photocatalysis 93 341-57
[11] Somorjai GA 1996 Modern Surface Science and Surface Technologies: An Introduction Chem. Rev 96 1223-36
[12] Yu JC, Ho W, Lin J, Yip H, Wong PK 2003 Photocatalytic Activity, Antibacterial Effect, and Photoinduced Hydrophilicity of TiO2 Films Coated on a Stainless Steel Substrate Environ. Sci. Technol 37 2296-301
[13] Sunada K, Watanabe T, Hashimoto K 2003 Bactericidal Activity of Copper-Deposited TiO2 Thin Film under Weak UV Light Illumination Environ. Sci. Technol. 37 4785-89
[14] Liu H, Chen Q, Song L, Ye R, Lu J, Li H 2008 Ag-doped antibacterial porous materials with slow release of silver ions Journal of Non-Crystalline Solids 354 1314-17
[15] Yogi C, Kojima K, Wada N, Tokumoto H, Takai T, Mizoguchi T, Tamiaki H 2008 Photocatalytic degradation of methylene blue by TiO2 film and Au particles-Ti02 composite film Thin Solid Films 516 5881-84
[16] Doong RA, Chang WH 1997 Photoassisted titanium dioxide mediated degradation of organophosphorus pesticides by hydrogen peroxide Journal of Photochemistry and Photobiology a-Chemistry 107 239-44
[17] San N, Cinar Z 2002 Structure - Activity relations for the photodegradation reactions of monosubstituted anilines in Ti02 suspensions Journal of Advanced Oxidation Technologies 5 85-92
[18] San N, Hatipoglu A, Kocturk G, Cinar Z 2002 Photocatalytic degradation of 4-nitrophenol in aqueous TiO2 suspensions: Theoretical prediction of the intermediates Journal of Photochemistry and Photobiology a-Chemistry 146 189-97
[19] San N, Hatipoglu A, Kocturk G, Cinar Z 2001 Prediction of primary intermediates and the photodegradation kinetics of 3-aminophenol in aqueous Ti02 suspensions Journal of Photochemistry and Photobiology a-Chemistry 139 225-32
[24] St.meld. nr. 7 (2008-2009) - Et nyskapende og bærekraftig Norge. In Oslo, Norwegian Ministry of Trade and Industry, http://www.regjeringen.no/nb/dep/nhd/dok/regpubl/stmeld/2008-2009/stmeld-nr-7-2008-2009-.html?id=538010
[25] Teruhisa Ohno, Yuji Masaki, Seiko Hirayama, Michio Matsumura, TiO2-Photocatalyzed Epoxidation of 1-Decene by H2O2 under Visible Light, Journal of Catalysis, Volume 204, Issue 1, (2001), P. 163-168

## Claims

1. A composition for locally alleviating inflammation and/or for simultaneously preventing microbial growth and debriding a wound, said composition comprising nanoparticles of TiO₂ having a mean particle diameter (D₅₀) of 20-50 nm at a concentration of 3-10 g/L, preferably 4-8 g/L and H₂O₂ at a final concentration at 2.5-25 % by volume.

2. A composition according to claim 1, wherein the concentration of H₂O₂ is 3-25 % by volume, preferably 4-20 % by volume, more preferably 5-15 % by volume.

3. A composition according to claim 1 or 2, wherein said nanoparticles of TiO₂ have a mean particle diameter (D₅₀) of 20-30 nm, such as 20, 21, 22, 23, 24, 25, 26, 27, 28, 29 and 30 nm.

4. A composition according to claim 1 comprising nanoparticles of TiO₂ having a mean particle diameter (D₅₀) of 20-50 nm at a concentration of 4-8 g/L, and hydrogen peroxide at a concentration of 5-15 % by volume.

5. A composition according to any one of the preceding claims, wherein said nanoparticles of TiO₂ has 0.01-5 atomic weight% fluorine in the surface layer.

6. A composition according to any one of claims 1-5 for medical use.

7. Use of a composition according to any one of claims 1-5, for the manufacture of a pharmaceutical composition for preventing microbial growth.

8. A composition according to any one of claims 1-5 for use for preventing microbial growth.

9. Use of a composition comprising nanoparticles of TiO₂ having a mean particle diameter (D₅₀) of 3-150 nm at a concentration of 1-2000 g/L and H₂O₂ at a final concentration a 2.5-25 % by volume or a composition as defined in any one of claims 1-5, for the manufacture of a pharmaceutical composition for wound debridement, for locally alleviating inflammation and/or for simultaneously preventing microbial growth and debriding a wound.

10. A composition as defined in any one of claims 1-5 for use for wound debridement, for use for locally alleviating inflammation and/or for use for simultaneously preventing microbial growth and debriding a wound.

11. A medical product, such as a crème or ointment, or device and/or cosmetic product or device comprising a composition as defined in any one of claims 1-5.

12. A wet wound dressing, a wound debridement product and/or a shampoo or a deodorant comprising a composition as defined in any one of claims 1-5.

13. A kit comprising a first container comprising nanoparticles of TiO₂ having a mean particle diameter (D₅₀) of 20-50 nm and a second container comprising H₂O₂ which nanoparticles of TiO₂ when mixed with said H₂O₂ provides a composition as defined in any one of claims 1-5.

14. A method for preparing a composition according to any one of claims 1-5, comprising the steps of mixing said nanoparticles of TiO₂ with said H₂O₂.

## Patentansprüche

1. Zusammensetzung zur lokalen Linderung von Entzündung und/oder zum gleichzeitigen Verhindern von mikrobiellem Wachstum und Debriding einer Wunde, wobei die Zusammensetzung Nanopartikel von TiO₂, die einen mittleren Partikeldurchmesser (D₅₀) von 20-50 nm haben, in einer Konzentration von 3-10 g/l, vorzugsweise 4-8 g/l, und H₂O₂ mit einer Endkonzentration von 2,5-25 Vol-% umfasst.

2. Zusammensetzung gemäß Anspruch 1, wobei die Konzentration von H₂O₂ 3-25 Vol.-%, vorzugsweise 4-20 Vol.-%, bevorzugter 5-15 Vol.-%, ist.

3. Zusammensetzung gemäß Anspruch 1 oder 2, wobei die Nanopartikel von TiO₂ einen mittleren Partikeldurchmesser (D₅₀) von 20-30 nm, z.B. 20, 21, 22, 23, 24, 25, 26, 27, 28, 29 und 30 nm, haben.

4. Zusammensetzung gemäß Anspruch 1, die Nanopartikel von TiO₂, die einen mittleren Partikeldurchmesser (D₅₀) von 20-50 nm haben, in einer Konzentration von 4-8 g/l und Wasserstoffperoxid in einer Konzentration von 5-15 Vol.-% umfasst.

5. Zusammensetzung gemäß einem der vorangehenden Ansprüche, wobei die Nanopartikel von TiO₂ 0,01-5 Atomgewichts-% Fluor in der Oberflächenschicht haben.

6. Zusammensetzung gemäß einem der Ansprüche 1 bis 5 zur medizinischen Verwendung.

7. Verwendung einer Zusammensetzung gemäß einem der Ansprüche 1 bis 5 zur Herstellung einer pharmazeutischen Zusammensetzung zur Verhinderung von mikrobiellem Wachstum.

8. Zusammensetzung gemäß einem der Ansprüche 1 bis 5 zur Verwendung bei der Verhinderung von mikrobiellem Wachstum.

9. Verwendung einer Zusammensetzung, die Nanopartikel von TiO₂, die einen mittleren Partikeldurchmesser (D₅₀) von 3-150 nm haben, in einer Konzentration von 1-2000 g/l und H₂O₂ in einer Endkonzentration von 2,5-25 Vol.-% umfasst, oder einer Zusammensetzung, wie sie in einem der Ansprüche 1 bis 5 definiert ist, für die Herstellung einer pharmazeutischen Zusammensetzung zum Wund-Debridement, für lokale Linderung von Entzündung und/oder zur gleichzeitigen Verhinderung von mikrobiellem Wachstum und Debriding einer Wunde.

10. Zusammensetzung, wie sie in einem der Ansprüche 1 bis 5 definiert ist, zur Verwendung zum Wund-Debridement, zur Verwendung zur lokalen Linderung von Entzündung und/oder zur Verwendung zur gleichzeitigen Verhinderung von mikrobiellem Wachstum und Debriding einer Wunde.

11. Medizinisches Produkt, zum Beispiel Creme oder Salbe, oder Vorrichtung und/oder kosmetisches Produkt oder Vorrichtung, umfassend eine Zusammensetzung, wie sie in einem der Ansprüche 1 bis 5 definiert ist.

12. Feuchter Wundverband, Wunden-Debridement-Produkt und/oder Shampoo oder Deodorant, umfassend eine Zusammensetzung, wie sie in einem der Ansprüche 1 bis 5 definiert ist.

13. Kit, umfassend einen ersten Behälter, der Nanopartikel von TiO₂, die einen mittleren Partikeldurchmesser (D₅₀) von 20-50 nm haben, umfasst, und einen zweiten Behälter, der H₂O₂ umfasst, wobei Nanopartikel von TiO₂, wenn sie mit dem H₂O₂ gemischt werden, eine Zusammensetzung, wie sie in einem der Ansprüche 1 bis 5 definiert ist, bereitstellen.

14. Verfahren zur Herstellung einer Zusammensetzung gemäß einem der Ansprüche 1 bis 5, umfassend die Schritte des Mischens der Nanopartikel von TiO₂ mit dem H₂O₂.

## Revendications

1. Composition pour le soulagement local d'une inflammation et/ou pour la prévention de manière simultanée d'une croissance microbienne et du débridement d'une plaie, ladite composition comprenant des nanoparticules de TiO₂ ayant un diamètre de particule moyen (D₅₀) de 20 à 50 nm à une concentration de 3 à 10 g/L, de préférence de 4 à 8 g/L et de l'H₂O₂ à une concentration finale de 2,5 à 25 % en volume.

2. Composition selon la revendication 1, dans laquelle la concentration en H₂O₂ est de 3 à 25 % en volume, de préférence de 4 à 20 % en volume, plus préférablement de 5 à 15 % en volume.

3. Composition selon la revendication 1 ou 2, dans laquelle lesdites nanoparticules de TiO₂ ont un diamètre de particule moyen (D₅₀) de 20 à 30 nm, tel que 20, 21, 22, 23, 24, 25, 26, 27, 28, 29 et 30 nm.

4. Composition selon la revendication 1, comprenant des nanoparticules de TiO₂ ayant un diamètre de particule moyen (D₅₀) de 20 à 50 nm à une concentration de 4 à 8 g/L, et du peroxyde d'hydrogène à une concentration de 5 à 15 % en volume.

5. Composition selon l'une quelconque des revendications précédentes, dans laquelle lesdites nanoparticules de TiO₂ comprennent de 0,01 à 5 % en poids atomique de fluor dans la couche superficielle.

6. Composition selon l'une quelconque des revendications 1 à 5, pour son utilisation dans le domaine médical.

7. Utilisation d'une composition selon l'une quelconque des revendications 1 à 5, pour la fabrication d'une composition pharmaceutique pour la prévention d'une croissance microbienne.

8. Composition selon l'une quelconque des revendications 1 à 5, pour son utilisation dans la prévention d'une croissance microbienne.

9. Utilisation d'une composition comprenant des nanoparticules de TiO₂ ayant un diamètre de particule moyen (D₅₀) de 3 à 150 nm à une concentration de 1 à 2000 g/L et de l'H₂O₂ à une concentration finale de 2,5 à 25 % en volume ou d'une composition telle que définie à l'une quelconque des revendications 1 à 5, pour la fabrication d'une composition pharmaceutique pour le débridement d'une plaie, pour le soulagement local d'une inflammation et/ou pour la prévention de manière simultanée d'une croissance microbienne et du débridement d'une plaie.

10. Composition telle que définie à l'une quelconque des revendications 1 à 5, pour son utilisation pour le débridement d'une plaie, pour son utilisation pour le soulagement local d'une inflammation et/ou pour son utilisation pour la prévention de manière simultanée d'une croissance microbienne et du débridement d'une plaie.

11. Produit médical, tel qu'une crème ou une pommade, ou dispositif et/ou produit cosmétique ou dispositif comprenant une composition telle que définie à l'une quelconque des revendications 1 à 5.

12. Pansement humide, produit de débridement d'une plaie et/ou shampooing ou déodorant comprenant une composition telle que définie à l'une quelconque des revendications 1 à 5.

13. Kit comprenant un premier contenant comprenant des nanoparticules de TiO₂ ayant un diamètre de particule moyen (D₅₀) de 20 à 50 nm et un second contenant comprenant de l'H₂O₂, lesquelles nanoparticules de TiO₂, lorsqu'elles sont mélangées avec ledit H₂O₂, donnent une composition telle que définie à l'une quelconque des revendications 1 à 5.

14. Procédé de préparation d'une composition selon l'une quelconque des revendications 1 à 5, comprenant les étapes de mélange desdites nanoparticules de TiO₂ avec ledit H₂O₂.
